# EUROPÄISCHE PATENTSCHRIFT

(11) **EP 1 374 829 B1**
(45) Veröffentlichungstag und Bekanntmachung des Hinweises auf die Patenterteilung: **02.11.2005**
(21) Anmeldenummer: 03014628.6
(22) Anmeldetag: 26.06.2003
(51) Int. Cl.: A61K 6/083, A61K 6/00, C07F 9/38

(54) **Dentalmaterialien auf der Basis von Acrylesterphosphonsäuren**
Dental material on the basis of acryloester phosphonic acid
Matériaux dentaires à la base d'ester d'acrylique et d'acide phosphonique

(30) Priorität: 26.06.2002 DE 10228540; 26.07.2002 DE 10234326
(43) Veröffentlichungstag der Anmeldung: 02.01.2004
(73) Patentinhaber: Ivoclar Vivadent AG, 9494 Schaan (LI)
(72) Erfinder: Moszner, Norbert, Prof. Dr., FL-9495 Triesen (LI); Salz, Ulrich, Dr., 88131 Lindau (DE); Zeuner, Frank, Dr., 9490 Vaduz (LI); Zimmermann, Jörg, 6890 Lustenau (AT); Rheinberger, Volker, Dr., 9490 Vaduz (LI)
(74) Vertreter: UEXKÜLL & STOLBERG

(56) Entgegenhaltungen:
- EP-A- 1 148 060
- EP-A- 1 222 910
- DE-A- 19 746 708

## Beschreibung

Die vorliegende Erfindung betrifft Acrylesterphosphonsäuren, die eine hohe Hydrolysestabilität aufweisen und sich insbesondere zur Herstellung von Dentalmaterialien eignen.

Polymerisationsfähige Phosphonsäuren sind vor allem als Comonomere von polymerchemischer Bedeutung und gestatten die Herstellung von organischen Polymeren mit hoher thermischer Stabilität, guten Hafteigenschaften, geringer Entflammbarkeit und guter Löslichkeit in polaren Lösungsmitteln. Für diese Zweck sind zahlreiche monomere Phosphonsäuren mit polymerisationsfähigen Vinyl-, Dienyl-, Allyl- oder Styrylgruppen synthetisiert und polymerisiert worden. Eine Übersicht zu Phosphonsäuren gibt Houben-Weyl, Methoden der Organischen Chemie, Band E 20 (2.Teil), G. Thieme Verlag, Stuttgart-New York 1987, 1300 ff. Beispiele für solche konventionellen polymerisierbaren Phosphonsäuren sind Vinylphosphonsäure, Allylbenzolphosphonsäure, α-Aminoallylphoshonsäure, 1,3-Butadien- oder Isoprenphosphonsäure, 4-Vinylbenzolphosphonsäure oder 2-(4-Vinylphenyl)-ethanphosphonsäure.

Phosphonsäuren, bei denen die C=C-Gruppierung direkt oder über ein Sauerstoffatom an das Phosphoratom gebunden ist, wie z.B. in Vinylphosphonsäure bzw. Ethylphosphonsäuremonovinylester, weisen nur eine geringe Neigung zur Homopolymerisation auf, so daß nur Homopolymere mit einer geringen Molmasse erhalten werden können.

Aus der DE 199 18 974 A1 sind Dentalwerkstoffe auf der Basis von polymerisierbaren (Meth)acrylsäurederivaten von Phosphonsäuren, wie z.B. 2-Methacryloyloxyethylphosphonsäure, bekannt bei denen die polymerisationsfähige (Meth)acrylgruppe über einen Alkylenrest an den Phosphor gebunden ist. Diese Verbindungen lassen sich gut polymerisieren, sind jedoch nicht hydrolysestabil.

Die DE 197 46 708 A1 offenbart polymerisierbare Acrylphosphonsäuren, die in wäßriger Lösung hydrolysestabil sind, über gute Hafteigenschaften verfügen, mit herkömmlichen radikalischen Initiatoren polymerisiert werden können und sich daher besonders als Bestandteil von Dentalmaterialien eignen. Die Acrylphosphonsäuren zeigen in Form ihrer Carbonsäureester eine gute Löslichkeit in Wasser und polaren organischen Lösungsmitteln während sie in Form der Carbonsäuren zwar gut in Wasser aber kaum in organischen Lösungsmitteln löslich sind. Das unterschiedliche Lösungsverhalten von Ester und Säure kann bei wasserhaltigen Materialien nachteilig sein. Die Hydrolyse der Carbonsäureester zur freien Carbonsäure unter Abspaltung von Alkohol kann die Löslichkeit der Monomeren signifikant verändern und so zur partiellen oder vollständigen Ausfällung der Phosphonsäurekomponente führen und damit die Eigenschaften des Materials beeinflussen.

Die DE 100 18 968 offenbart Dentalmaterialien auf der Basis von hydrolysestabilen Acrylamid- und Acrylnitrilphosphonsäuren.

A.M. Kawamoto, M.M. Campbell, J. Chem. Soc., Perkin Trans. 1, 1997, 1249 beschreiben Difluorvinylphosphonatanaloga von Phosphoenolpyruvat, die potentielle Inhibitoren des Shikimisäurewegs darstellen sollen.

Schließlich sind aus der EP 1 169 996 A1 Phosphonsäurederivate bekannt, bei denen polymerisierbare Gruppen über eine Amidgruppe an eine Phosphonsäuregruppe gebunden sind, wie beispielsweise in 4-Methacrylamido-4-methyl-pentylphosphonsäure. Diese Verbindungen weisen nur eine geringe radikalische Polymerisationsfähigkeit auf.

Der Erfindung liegt die Aufgabe zugrunde, polymerisationsfähige Acrylphosphonsäuren bereitzustellen, die sich gegenüber bekannten Acrylphosphonsäuren durch ein weiter verbesserte Hydrolysebeständigkeit auszeichnen.

Erfindungsgemäß wird diese Aufgabe durch Acrylesterphosphonsäuren der allgemeinen Formel (I), Stereoisomeren davon und Mischungen von diesen gelöst, 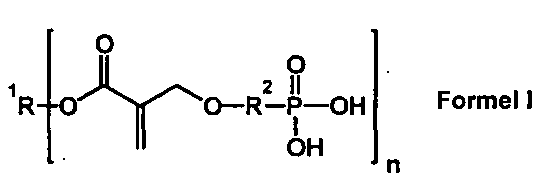 in der
- n: 1 oder 2 ist,
mit der Maßgabe, daß für n = 1 R¹ die Bedeutung 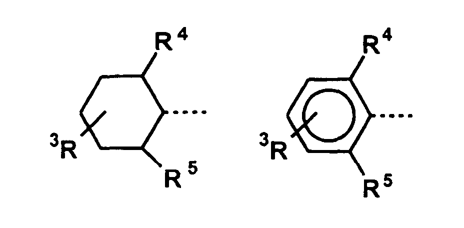 und für n = 2 R¹ die Bedeutung 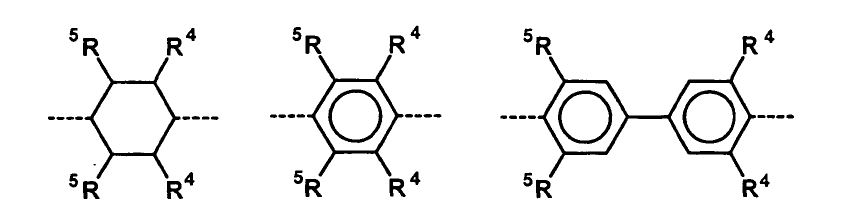 hat;
- R²: ein C₁ bis C₁₂-Alkylenrest, C₄-C₈-Cycloalkylenrest oder C₇- bis C₁₅-Alkylenphenylenrest ist;
- R³: Wasserstoff, ein C₁- bis C₅-Alkylrest oder ein C₁- bis C₅-O-Alkylrest ist; und
- R⁴, R⁵: unabhängig voneinander jeweils für einen C₁- bis C₅-Alkylrest oder einen C₁- bis C₅-O-Alkylrest stehen.

Die einzelnen Alkyl- und Alkylenreste können dabei geradkettig, verzweigt oder cyclisch sein.

Die einzelnen Reste R2, R3, R4 und/oder R5 können unsubstituiert oder durch einen oder mehrere Substituenten, wie Cl, Br, CH₃O, OH, COOH, CN, =O, =S, =NR⁶ oder -NR⁷-CO-C(=CH₂)CH₂-Y-R⁸-PO(OH)₂, vorzugsweise Cl, CH₃O, OH, COOH, =O oder =NR⁶ substituiert sein, wobei R⁶ und R⁷ unabhängig voneinander jeweils für Wasserstoff, einen geradkettigen oder verzweigten C₁- bis C₁₀-Alkyl- oder C₆- bis C₁₀-Arylrest, vorzugsweise für Wasserstoff oder einen geradkettigen C₁- bis C₃-Alkylrest stehen und R⁸ ein geradkettiger oder verzweigter C₁- bis C₁₀-Alkylen- oder C₆- bis C₁₄-Arylen-Rest, vorzugsweise ein geradkettiger oder verzweigter C₁- bis C₅-Alkylenrest oder Phenylen ist.

Die Erfindung betrifft ferner die Verwendung dieser hydrolysestabilen Acrylesterphosphonsäuren zur Herstellung von Polymeren, Adhäsiven, Dentalmaterialien oder anderen Materialien und Werkstoffen. Gegenstand der vorliegenden Erfindung sind ebenfalls die Verwendung der Acrylesterphosphonsäuren als Komponente von Adhäsiven, Zementen, Kompositen, Formkörpern oder Dentalmaterialien sowie Polymere oder Copolymere, die durch Homo- oder Copolymerisation der Acrylesterphosphonsäuren erhältlich sind.

Für die oben angegebenen Variablen der Formel (I) existieren die folgenden bevorzugte Definitionen, die, sofern nicht anders angegeben, unabhängig voneinander gewählt werden können:
- n =: 1,
- R¹ =: 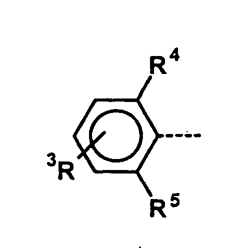
- R² =: ein C₁ bis C₆-Alkylenrest;
- R³ =: Wasserstoff, ein C₁- bis C₃-Alkylrest; und
- R⁴, R⁵ =: unabhängig voneinander jeweils ein C₁- bis C₃-Alkylrest.

Bevorzugte Verbindungen sind demgemäß solche, bei denen mindestens eine der Variablen der Formel (I) die vorstehend beschriebene bevorzugte Definition aufweist. Besonders bevorzugte erfindungsgemäße Monomere sind solche bei denen alle Variablen eine der bevorzugten Bedeutungen haben.

Die erfindungsgemäßen Acrylesterphosphonsäuren der Formel (I) lassen sich durch Veresterung von entsprechenden OHgruppenhaltigen mono- (n = 1) oder difunktionellen (n = 2) Verbindungen **R**^{**1**}**-(OH)**_{**n**} mit geeigneten COOH-gruppenhaltigen Acrylatetherphosphonsäureestern **AEPE** analog nach den aus der Literatur bekannten Methoden zur Herstellung von Carbonsäureestern (vgl. u.a. B. Neises, W. Steglich, Angew. Chem. **90** (1978) 556, oder A Hassner, V. Alexanian, Tetrahedron Lett. **46** (1978) 4475) in Gegenwart von Dicyclohexylcarbodiimid (DCC) und 4-Dimethylaminopyridin (**DMAP**), hydrolytische Abspaltung der Schutzgruppen (**SG**) durch Silylierung mit Trialkylsilanen, z.B. Trimethylsilylchlorid/NaI oder -bromid (**TMSBr**), und nachfolgender Umsetzung mit Alkoholen, wie z.B. Methanol, oder Wasser (S. Freeman, J. Chem . Soc., Perkin Trans. 2 (1991) 263.) herstellen.

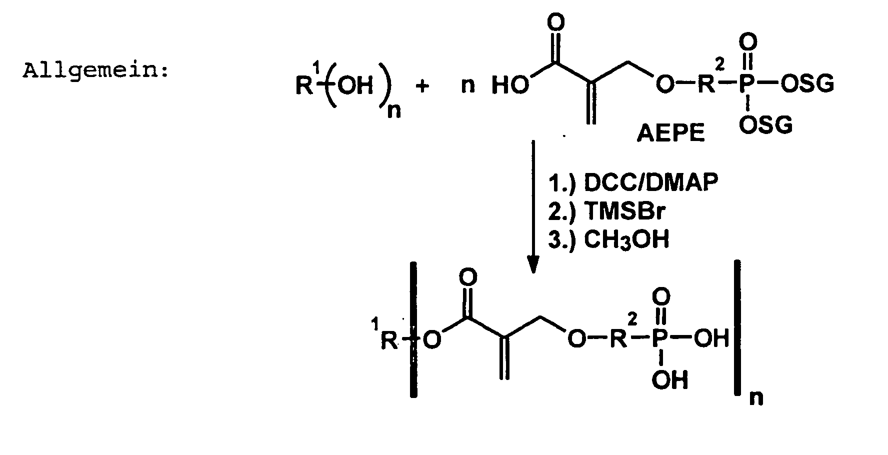

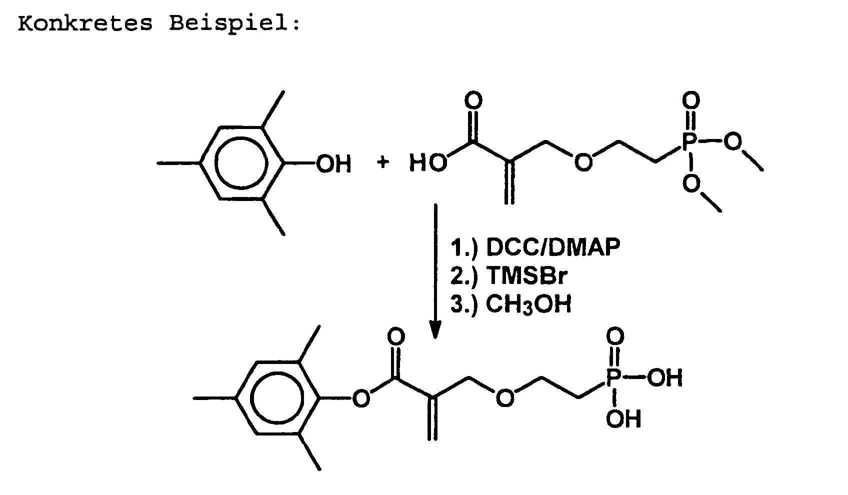

Dabei können die geeigneten COOH-gruppenhaltigen Acrylatetherphosphonsäureester **AEPE** aus den entsprechenden Carbonsäureestern durch partielle Hydrolyse, z.B. mit einer äquimolaren Menge an Lauge bei -5 °C analog zur Literatur (vgl. N. Moszner, F. Zeuner, U. K. Fischer, V. Rheinberger, Macromol. Chem. Phys. **200** (1999) 1062), synthetisiert werden.

Bevorzugte Beispiele für die erfindungsgemäßen Acrylesterphosphonsäuren der Formel (**I**) sind:

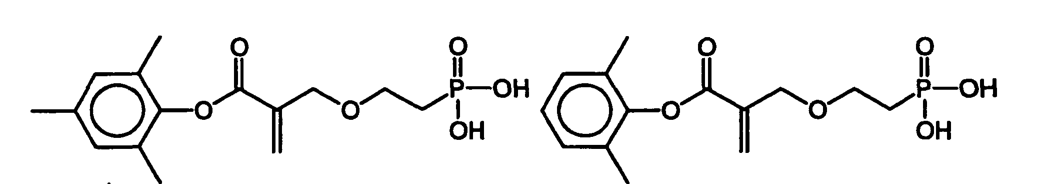

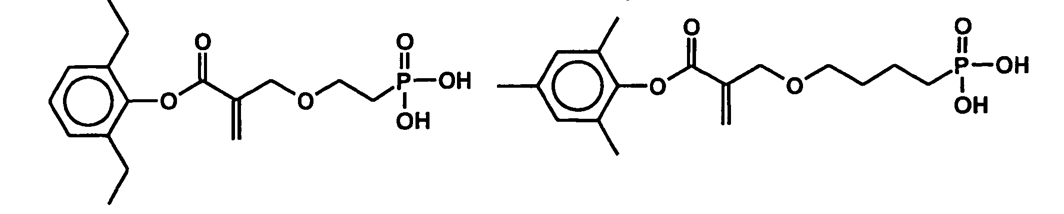

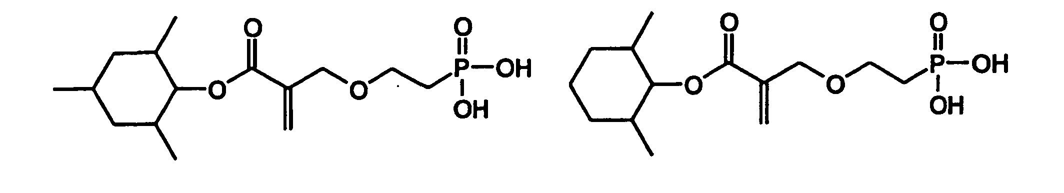

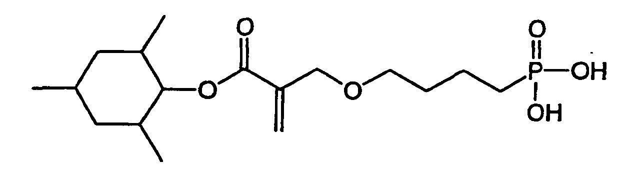

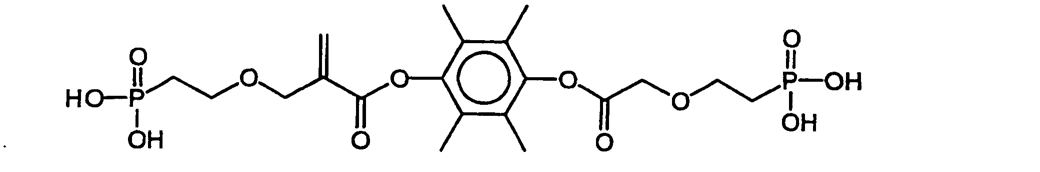

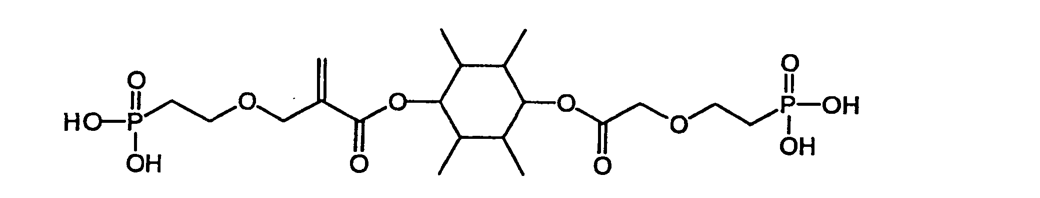

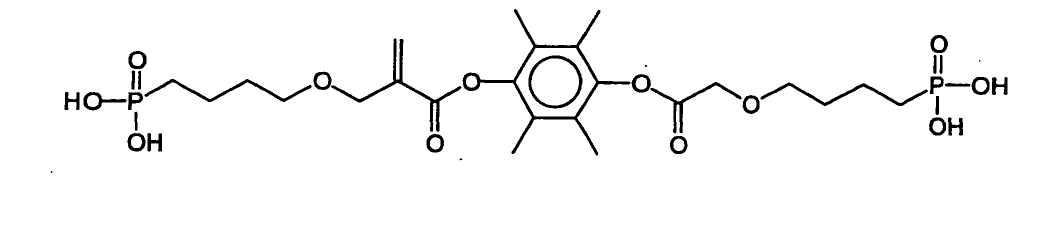

Die erfindungsgemäßen Acrylesterphosphonsäuren sind stark sauer und sehr gut in Wasser oder Mischungen von Wasser mit polaren Lösungsmitteln, wie Aceton, Ethanol, Acetonitril oder Tetrahydrofuran (THF) löslich. In Ihnen ist die acide Phosphonsäuregruppe mit der polymerisationsfähigen Acrylatgruppe über eine hydrolysestabile Ethergruppe verbunden. Darüber hinaus weist auch die Estergruppe in den erfindungsgemäßen Acrylesterphosphonsäuren eine hoher Hydrolysestabilität auf, was zusammen genommen eine deutliche Verbesserung gegenüber herkömmlichen polymerisierbaren Acrylphosphonsäuren darstellt.

Im Zusammenhang mit der vorliegenden Erfindung werden solche Verbindungen als hydrolysestabil bezeichnet, die in Wasser oder in Mischungen von Wasser und wassermischbaren Lösungsmitteln bei einer Konzentration von ca. 20 Gew.-% und einem pH-Wert von ca. 2,0 bei 37 °C für mindestens 6 Wochen stabil sind, d.h. zu weniger als 1 % hydrolysieren.

Aufgrund des Vorliegens von polymerisierbaren Gruppen eignen sich die erfindungsgemäßen Acrylphosphonsäureester als Ausgangsmaterialien für die Herstellung von Polymeren und Copolymeren. Dabei lassen sie sich mit den bekannten Methoden der radikalischen Polymerisation homopolymerisieren oder z.B. mit geeigneten Comonomeren copolymerisieren.

Die erfindungsgemäßen Acrylesterphosphonsäuren können in freier Form oder in Form ihrer Salze, d.h. als Phosphonate oder Phosphonatester, eingesetzt werden, wobei im Fall der Salze als Gegenionen vorzugsweise Alkalimetallionen, insbesondere Natrium- und Lithiumionen, sowie organische Ammoniumionen dienen, insbesondere solche, die sich von Aminbeschleunigern, wie N,N-Dihydroxyethyl-p-toluidin, N,N-Bis-(2-hydroxy-3-methacryloxypropyl-3,5-xylidin oder 4-(Dimethylamino)-benzoesäure-2-ethylhexylester, ableiten. Aminbeschleuniger werden im Dentalbereich als Komponente beispielsweise von Photoinitiatorsystemen eingesetzt. Es handelt sich im allgemeinen um tert. Amine, die als H-Donatoren wirken können und damit die Radikalbildung beschleunigen (vgl. L.A. Linden, "Photocuring of Polymeric Dental Materials and Plastic Composite Resins" in Radiation Curing in Polymer Science and Technology, Vol. IV, J.P. Fouassier, J.F. Rabek (Herausgeber), Elsevier Appl. Sci., London, Ney York 1993, 396f.).

Zur Durchführung der Polymerisation können die bekannten radikalischen Initiatoren (vgl. Encyclopedia of Polymer Science and Engineering, Vol. 13, Wiley-Interscience Publisher, New York 1988, 754 ff.) eingesetzt werden.

Zur Initiierung der radikalischen Photopolymerisation werden vorzugsweise Benzophenon, Benzoin sowie deren Derivate oder α-Diketone oder deren Derivate wie 9,10-Phenanthrenchinon, 1-Phenyl-propan-1,2-dion, Diacetyl oder 4,4-Dichlorbenzil eingesetzt. Bevorzugt werden Campherchinon und 2,2-Methoxy-2-phenyl-acetophenon und besonders bevorzugt α-Diketone in Kombination mit Aminen als Reduktionsmittel, wie z.B. 4-(Dimethylamino)-benzoesäureester, N,N-Dimethylaminoethylmethacrylat, N,N-Dimethyl-sym.-xylidin oder Triethanolamin, verwendet.

Als thermische Initiatoren eignen sich besonders Azoverbindungen, wie Azobis(isobutyronitril) (AIBN) oder Azobis-(4-cyan-valeriansäure) oder Peroxide, wie Dibenzoylperoxid, Dilauroylperoxid, tert.-Butylperoctoat, tert.-Butylperbenzoat oder Di-(tert.-butyl)-peroxid. Als Initiatoren für die Heißhärtung eignen sich auch Benzpinakol und 2,2'-Dialkylbenzpinakole. Als Initiatoren für eine bei Raumtemperatur durchgeführte Polymerisation werden Redox-Initiatorkombinationen, wie z.B. Kombinationen von Benzoylperoxid mit N,N-Dimethylsym.-xylidin oder N,N-Dimethyl-p-toluidin, verwendet. Darüber hinaus sind auch Redoxsysteme bestehend aus Peroxiden und solchen Reduktionsmittel, wie z.B. Ascorbinsäure, Barbiturate oder Sulfinsäuren, besonders geeignet.

Gegenstand der Erfindung sind auch Zusammensetzungen, die eine oder mehrere Acrylesterphosphonsäuren gemäß Formel (I) und außerdem auch einen Initiator für die radikalische Polymerisation enthalten.

Aufgrund der Hydrolysestabilität der erfindungsgemäßen Acrylesterphosphonsäuren sind die Zusammensetzungen bei Raumtemperatur auch in Gegenwart von Wasser lagerstabil und eignen sich besonders als Adhäsive oder Zemente vor allem für dentale Anwendungen.

Weiter bevorzugt sind Zusammensetzungen, die neben Acrylesterphosphonsäure und ggf. Initiator zusätzlich ein oder mehrere radikalisch polymerisierbare Monomere enthalten.

Als Comonomere eignen sich monofunktionelle und/oder mehrfunktionelle radikalisch polymerisierbare Monomere, insbesondere difunktionelle Vernetzermonomere. Unter monofunktionellen Monomeren werden Verbindungen mit einer, unter mehrfunktionellen Monomeren Verbindungen mit zwei und mehr radikalisch polymerisierbaren Gruppen verstanden. Für die Herstellung von Adhäsiven oder Dentalmaterialien eignen sich vor allem vernetzende bi- oder mehrfunktionelle Acrylate oder Methacrylate, wie z.B. Bisphenol-A-di(meth)acrylat, Bis-GMA (das Additionsprodukt von Methacrylsäure und Bisphenol-A-diglycidylether), UDMA (das Additionsprodukt von Hydroxyethylmethacrylat und 2,2,4-Trimethylhexamethylendiisocyanat), Di-, Tri- oder Tetraethylenglykoldi(meth)acrylat, Trimethylolpropantri(meth)acrylat und Pentaerythrittetra(meth)-acrylat. Es eignen sich ebenfalls die durch Veresterung von (Meth)acrylsäure mit den entsprechenden Diolen zugänglichen Verbindungen Butandioldi(meth)acrylat, 1,10-Decandioldi(meth)-acrylat und 1,12-Dodecandioldi(meth)acrylat.

Besonders bevorzugte radikalisch polymerisierbare Monomere sind Acrylamide oder Hydroxyalkylacrylamide.

Auf Grund ihrer Hydrolysestabilität sind Amide der allgemeinen Formel BXₙ besonders bevorzugt, in der
- B: für einen n-fach mit der Gruppe X' substituierten Kohlenwasserstoffrest mit 1 bis 50 Kohlenstoffatomen steht, der eine oder mehrere der Gruppen O, S, NH, CO-NH, NH-CO, NH-CO-O, O-CO-NH und/oder NH-CO-NH enthalten kann,
- X': für die Gruppe 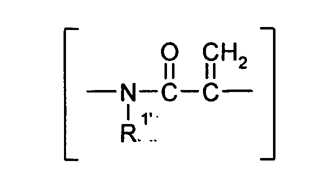 steht, die über das Stickstoffatom oder über C-2 an den Rest B gebunden ist, wobei die nicht mit B verbundene Bindungsstelle einen Rest R^{2'} trägt,
- R^{1'}: Wasserstoff, eine Alkylgruppe mit 1 bis 20 Kohlenstoffatomen oder ein Phenylrest ist, wobei sich zwei oder mehr Reste X' einen Rest R^{1'} teilen können und wobei R^{1'} auch Bestandteil des Restes B sein kann,
- R^{2'}: Wasserstoff, eine Alkylgruppe mit 1 bis 20 Kohlenstoffatomen oder ein Phenylrest ist, und
- n': eine Zahl von 2 bis 5 ist.

Bei der Gruppe X' handelt es sich um N-substituierte Amidgruppen, die über den Amidstickstoff oder über das Kohlenstoffatom C-2 an den Reste B gebunden sind.

Amide diesen Typs, bevorzugte Derivate davon und deren Herstellung werden in der DE 101 01 523 offenbart.

Weiter bevorzugt sind Hydroxyalkylacrylamide der Formel 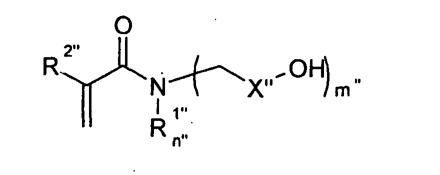 in der
- X": für einen C₁- bis C₁₂-Alkylenrest oder C₁- bis C₁₅-Alkylenphenylenrest, vorzugsweise für einen C₁- bis C₁₀-Alkylenrest und ganz besonders bevorzugt für einen C₁- bis C₈-Alkylenrest steht,
- R^{1"}: für einen C₁- bis C₁₀-Alkylrest, Phenyl oder Wasserstoff steht, vorzugsweise für einen C₁- bis C₆-Alkylrest oder Wasserstoff, besonders bevorzugt einen C₁- bis C₃-Alkylrest oder Wasserstoff steht,
- m",n": unabhängig voneinander 0, 1 oder 2 sind, wobei m" + n" gleich 2 ist, und
- R^{2"}: für Wasserstoff, Methyl oder X'''-OH steht, wenn m" größer oder gleich 1 ist, oder für X'''-OH steht, wenn m" gleich 0 ist, wobei X''' eine der für X" angegebenen Bedeutungen hat und vorzugsweise C₁- bis C₆-Alkylen, besonders bevorzugt C₁- bis C₃-Alkylen ist, und
wobei für n" = 2 die beiden Reste R^{1"} und für m" = 2 die beiden Reste -X"- gleich oder verschieden sein können. Die obigen Hydroxyalkylacrylamide enthalten keine Aldehydgruppen.

Bei den genannten Alkyl- und Alkylenresten handelt es sich vorzugsweise um lineare Gruppen. Verbindungen, bei denen m" und n" jeweils gleich 1 sind, sind besonders bevorzugt. Weiter bevorzugt sind Verbindungen, die 1, 2 oder 3 Hydroxylgruppen pro Molekül aufweisen.

Hydroxyalkylamide diesen Typs, bevorzugte Derivate davon und deren Herstellung werden in der DE 102 28 540 beschrieben.

Bevorzugte monofunktionelle radikalisch polymerisierbar Monomere, die sich besonders als Verdünnermonomere eignen, sind hydrolysestabile Mono(meth)acrylate, wie z.B. Mesitylmethacrylat, oder 2-(Alkoxymethyl)acrylsäuren, wie z.B. 2-(Ethoxymethyl)acrylsäure, 2-(Hydroxymethyl)acrylsäure, N-mono- oder -disubstitiuierte Acrylamide, wie z.B. N-Ethylacrylamid, N,N-Dimethacrylamid, N-(2-Hydroxyethyl)acrylamid oder N-(2-Hydroxyethyl)-N-methyl-acrylamid, sowie N-monosubstituierte Methacrylamide, wie z.B. N-Ethylmethacrylamid oder N-(2-Hydroxyethyl)methacrylamid und außerdem N-Vinylpyrrolidon oder Allylether.

Bevorzugte mehrfunktionelle radikalisch polymerisierbar Monomere, die sich besonders als Vernetzermonomere eignen, sind hydrolysestabile Urethane aus 2-(Hydroxymethyl)acrylsäure und Diisocyanten, wie z.B. 2,2,4-Trimethylhexamethylendiisocyanat oder Isophorondiisocyanat, vernetzende Pyrrolidone, wie z.B. 1,6-Bis(3-vinyl-2-pyrrolidonyl)-hexan, oder kommerziell zugängliche Bisacrylamide wie Methylen- oder Ethylenbisacrylamid, bzw. Bis(meth)acrylamide, wie z.B. N,N'-Diethyl-1,3-bis(acrylamido)-propan, 1,3-Bis(methacrylamido)-propan, 1,4-Bis(acrylamido)-butan oder 1,4-Bis(acryloyl)-piperazin, die durch Umsetzung aus den entsprechenden Diaminen mit (Meth)acrylsäurechlorid synthetisiert werden können.

Die Zusammensetzungen können eines oder mehrere der genannten Comonomere enthalten. Bevorzugt sind Zusammensetzungen, die mindestens ein mehrfunktionelles radikalisch polymerisierbares Monomer enthalten.

Weiterhin können die erfindungsgemäßen Zusammensetzungen zur Verbesserung der mechanischen Eigenschaften oder zur Einstellung der Viskosität mit organischen oder anorganischen Partikeln gefüllt werden. Bevorzugte anorganische partikuläre Füllstoffe sind amorphe kugelförmige Materialien auf der Basis von Oxiden, wie ZrO₂ und TiO₂ bzw. Mischoxiden aus SiO₂, ZrO₂ und/oder TiO₂, nanopartikuläre oder mikrofeine Füllstoffe, wie pyrogene Kieselsäure oder Fällungskieselsäure sowie Minifüllstoffe, wie Quarz-, Glaskeramik- oder Glaspulver mit einer durchschnittlichen Teilchengröße von 0,01 bis 1 µm sowie röntgenopake Füllstoffe, wie Ytterbiumtrifluorid oder nanopartikuläres Tantal (V) -oxid bzw. Bariumsulfat.

Die erfindungsgemäßen Zusammensetzungen können weiterhin radikalisch polymerisierbare Phosphorsäurederivate enthalten. Bevorzugt sind hydrolysestabile Phosphorsäurederivate, wie (Meth)acrylamidoalkyldihydrogenphosphate, insbesondere 6-(N-Methacryloylamino)hexyl- und 2-(N-Methacryloylamino)etyhldihydrogenphosphat. Diese Verbindungen verbessern die Ätzwirkung der Zusammensetzung und eignen sich daher besonders zur Herstellung selbstätzender Dentalwerkstoffe, wie Adhäsive und Zemente. Alkyl steht hier für C₁- bis C₁₂-Alkyl, vorzugsweise für C₁- bis C₆-Alkyl.

Außerdem enthalten die erfindungsgemäßen Zusammensetzungen vorzugsweise auch ein Lösungsmittel, wie Ester, z.B. Ethylacetat, vorzugsweise Wasser, Ethanol, Aceton, Methylenchlorid, Acetonitril oder Mischungen davon. Bevorzugt sind Zusammensetzungen, die als Lösungsmittel Wasser oder ein wasserhaltiges Lösungsmittelgemisch enthalten.

Zudem können die erfindungsgemäßen Zusammensetzungen weitere Additive enthalten, z.B. Stabilisatoren, Aromastoffe, mikrobiocide Wirkstoffe, fluoridionenabgebende Additive, optische Aufheller, Weichmacher und/oder UV-Absorber.

Zusammensetzungen, die ausschließlich hydrolysestabile Komponenten entsprechend der obigen Definition enthalten, stellen eine ganz besonders bevorzugte Ausführungsform der Erfindung dar.

Die erfindungsgemäßen Zusammensetzungen eignen sich besonders zur Herstellung von Dentalmaterialien, wie Zementen, beispielsweise von selbsthaftenden Befestigungszementen, und insbesondere von Adhäsiven. Solche Adhäsive zeichnen sich durch eine sehr gute Haftung auf der Zahnhartsubstanz aus und sind unter feuchten Bedingungen hydrolysestabil.

Bevorzugte Dentalmaterialien enthalten die folgenden Komponenten (a), (b), (c), (d), (e) und/oder (f):
a) 0,5 bis 70 Gew.-%, bevorzugt 10 bis 60 Gew.-% und besonders bevorzugt 15 bis 50 Gew.-% Acrylesterphosphonsäure gemäß Formel (I),
b) 0,01 bis 15 Gew.-%, besonders bevorzugt 0,1 bis 8,0 Gew.-% Initiator für die radikalische Polymerisation,
c) 0 bis 80 Gew.-%, bevorzugt 0 bis 60 Gew.-% und besonders bevorzugt 10 bis- 50 Gew.-% radikalisch polymerisierbares Monomer,
d) 0 bis 95 Gew.-%, bevorzugt 0 bis 80 Gew.-%, besonders bevorzugt 10 bis 70 Gew.-% und ganz besonders bevorzugt 20 bis 60 Gew.-% Lösungsmittel,
e) 0 bis 50 Gew.-%, bevorzugt 0 bis 30 Gew.-%, besonders bevorzugt 0 bis 20 Gew.-% und ganz besonders bevorzugt 10 bis 20 Gew.-% (Meth)acrylamidoalkyl-dihydrogenphosphat,
f) 0 bis 75 Gew.-%, besonders bevorzugt - in Abhängigkeit von der Anwendung - 0 bis 20 Gew.-% (Adhäsiv) oder 20 bis 75 Gew.-% (Zement) Füllstoff.

Besonders bevorzugt sind Zusammensetzungen, die zumindest die Komponenten (a), (c) und (d) enthalten.

Die Erfindung wird im folgenden anhand von Beispielen näher erläutert.

### Beispiele

### Beispiel 1: Synthese von 2-[2-Dihydroxyphosphoryl)-ethoxymethyl]-acrylsäure-2,4,6-trimethylphenylester (MAPA)

### 1. Stufe: 2-[2-Dimethoxyphosphoryl)-ethoxymethyl]-acrylsäure-2,4,6-trimethyl-phenylester (MAPAME)

Zu einer Lösung aus 27,6 g (116 mmol) 2-[4-(Dimethoxyphosphoryl)-2-oxabutyl]-acrylsäure, die nach Literatur (N. Moszner, F. Zeuner, S. Pfeiffer, I. Schurte, V. Rheinberger, M. Drache, Macromol. Mater. Eng. **286** (2001) 225) durch partielle Hydrolyse von 2-[4-(Dimethoxyphosphoryl)-2-oxabutyl]-acrylsäureethylester hergestellt wurde, 1,33 g (11 mmol) 4-Dimethylaminopyridin und 15,0 g (116 mmol) Mesitol in 60 ml THF wurden portionsweise 24,5 g (128 mmol) N-(3-Dimethylaminopropyl)-N'-ethylcarbodiimid-hydrochlorid innerhalb von 3 h unter kräftigen Rühren zugegeben. Die Mischung wurde über Nacht bei Raumtemperatur weiterreagieren gelassen. Danach wurden die flüchtigen Bestandteile abgezogen und der zurückbleibende ölige Rückstand in 250 ml Methylenchlorid aufgenommen. Die Mischung wurde mit 0,5N Salzsäure ausgeschüttelt, neutral gewaschen und über wasserfreiem Natriumsulfat getrocknet. Schließlich wurden alle flüchtigen Bestandteile im Feinvakuum abgetrennt und das Rohprodukt mittels Säulenchromatographie gereinigt: Kieselgelsäule, Laufmittel THF/Toluol 1:1. Man erhält 11,9 g **MAPAME** (29 % Ausbeute) als farbloses Öl.

¹H-NMR (400 MHz, CDCl₃, ppm) : 2.10 (s, 6H, o-CH₃), 2,17 (dt, 2H, PCH₂), 2,27 (s, 3H, p-CH₃), 3,75 (d, 6H, =CH₃), 3,81 (m, 2H, PCH₂CH₂) , 4,34s, 2H, OCH₂C=), 6,07 und 6,56 (2s, 1H, =CH₂) und 6, 87 (s, 2H, CₐᵣH).

¹³C-NMR (CDCl₃, 100 MHz): 16.1 (OCₐᵣ. Cₐᵣ.CH₃), 20.8 (OCₐᵣ. Cₐᵣ. Cₐᵣ. CₐᵣCH₃), 24.8 (d, PCH₂CH₂, ¹*J*_{C,P} = 140.2 Hz), 52.3 (d, OCH₃, ²*J*_{C,P} = 6.5 Hz), 64.7 (PCH₂CH₂), 69.2 (OCH₂C=), 127.4 (C=CH₂) , 129.2 (Cₐᵣ.H), 129.7 (OC_{ar.}Cₐᵣ.CH₃), 135.4 (OCₐᵣ. Cₐᵣ. Cₐᵣ. Cₐᵣ. CH₃), 136.4 (C=CH₂), 145.7 (OC_{ar.}), 163.7 (C=O).

³¹P-NMR (CDCl₃, 162 MHz): = + 32.5.

### 2. Stufe: 2-[2-Dihydroxyphosphoryl)-ethoxymethyl]-acrylsäure-2,4,6-trimethyl-phenylester

Zu einer Mischung von 9,83 g (27,6 mmol) der Verbindung **MAPAME** und 2,7 mg Phenothiazin MEHQ wurden 10,56 g (69 mmol) Trimethylsilylbromid vorsichtig unter Argon zugetropft und 3 h bei 45 °C gerührt. Dann wurde die Mischung am Rotationsverdampfer eingeengt, mit 60 ml Methanol versetzt, über Nacht gerührt, im Feinvakuum eingeengt und bis zur Gewichtskonstanz getrocknet. Es blieben 8,77 g (97 % Ausbeute) eines sehr zähen Öles von **MAPA** zurück.

¹H-NMR (CDCl₃, 400 MHz): = 2.08 (s, 6H, o-CH₃), 2.18 (dt, 2H, PCH₂), 2.26 (s, 3H, p-CH₃) , 3.82 (m, 2H, PCH₂CH₂), 4.31 (s, 2H, OCH₂C=), 6.06, 6.55 (jeweils s, 1H, =CH₂), 6.86 (s, 2H, C_{ar.}H), 9.48 (br, 2H, POH).

¹³C-NMR (CDCl₃, 100 MHz): = 16.2 (OCₐᵣ. Cₐᵣ. CH₃), 20.8 (OCₐᵣ. Cₐᵣ. Cₐᵣ. Cₐᵣ. CH₃), 26.8 (d, PCH₂CH₂, ¹*J*_{C,P} = 143.6 Hz), 64.6 (PCH₂CH₂), 69.2 (OCH₂C=), 127.9 (C=CH₂), 129.2 (C_{ar.}H), 129.7 (OCₐᵣ. Cₐᵣ. CH₃), 135.4 (OCₐᵣ. Cₐᵣ. Cₐᵣ. Cₐᵣ. CH₃), 136.1 (C=CH₂), 145.7 (OCₐᵣ.), 163.8 (C=O).

³¹P-NMR (CDCl₃, 162 MHz) : = + 33.6.

### Beispiel 2: Untersuchung der Hydrolysestabilität von MAPA

Es wurde eine 20%ige Lösung von **MAPA,** stabilisiert mit 200 ppm 2,6-Di-t-butyl-4-methylphenol, in D₂O/EtOH-d₆ (1:1) hergestellt, diese bei 37 °C gelagert und ¹H-NMR-spektroskopisch untersucht. Nach einer Standzeit von 2 Monaten konnten keine Veränderungen des ¹H-NMR-Spektrums festgestellt werden.

### Beispiel 3: Herstellung chemisch härtender Adhäsive auf der Basis von MAPA

Zur Untersuchung der Dentinhaftung auf Rinderzahndentin wurden Adhäsive mit folgender Zusammensetzung (Angaben in Gew.-%) hergestellt:

**Tabelle 1**

| **Zusammensetzung und Haftwerte für dentale Adhäsive auf der Basis von MAPA** | | | | |
|---|---|---|---|---|
| MAPA | Comonomer | Wasser | Initiator | Haftwerte |
| 16 % | 17 % **HMA** ¹⁾ | 60 % | 7 % | 9,1 ± 2,1 MPa |
| 16 % | 17 % **MAHP** ²⁾ | 60 % | 7 % | 11,0 ± 3,5 MPa |
| 20 % | 13 % **HEMAM** ³⁾ | 60 % | 7 % | 11,0 ± 2,0 MPa |
| 16 % | 17 % **DEPBAM** ⁴⁾ | 60 % | 7 % | 8,8 ± 2,4 MPa |

| | | | | |
|---|---|---|---|---|
| 1) **HMA** = 2-(Hydroxymethyl)acrylsäure | | | | |
| 2) **MAHP** = 6-(N-Methacryloylamino)hexyl-dihydrogenphosphat | | | | |
| 3) **HEMAM** = N-(2-Hydroxyethyl)-N-methyl-acrylamid | | | | |
| 4) **DEPBAM** = N, N' -Diethyl-1,3-propylen-bisacrylamid | | | | |

Rinderzähne wurden so in Kunststoffzylinder eingebettet, daß sich das Dentin und der Kunststoff in einer Ebene befanden. Mit einem kleinen Pinsel wurde jeweils eine Schicht der obigen Adhäsive 15 s in die Dentinoberfläche einmassiert und mit einem Luftbläser leicht verblasen. Auf die Adhäsivschicht wurde ein mit einem selbsthärtenden Zement beschichteter, vorpolymerisierter Zylinder aus dentalem Kompositmaterial (Tetric® Ceram, Firma Ivoclar Vivadent AG) aufgebracht und 10 Minuten im Dunkeln ausgehärtet. Anschließend wurden die Prüfkörper 24 h bei 37 °C in Wasser gelagert und die Scherhaftfestigkeit gemäß der ISO-Richtlinie "ISO 1994-ISO TR 11405: Dental Materials Guidance on Testing of Adhesion to Tooth Structure" bestimmt. Die Ergebnisse sind in Tabelle 1 zusammengefaßt. Sie zeigen, daß die erfindungsgemäßen Acrylesterphosphonsäuren nicht nur hydrolysestabil sind sondern auch eine hohe Haftung zwischen Dentin und Kompositmaterial gewährleisten.

## Patentansprüche

1. Acrylesterphosphonsäure der allgemeinen Formel (I), Stereoisomere davon und Mischungen von diesen, 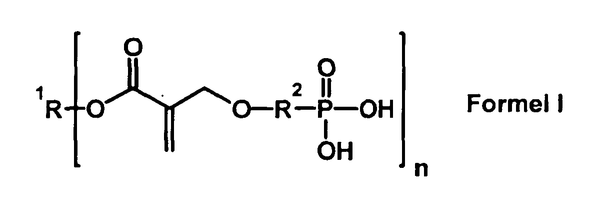 in der
n 1 oder 2 ist,
mit der Maßgabe, daß
für n = 1 R¹ die Bedeutung 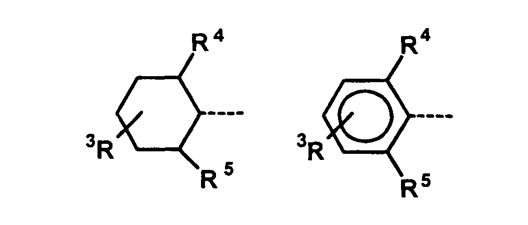 und für n = 2 R¹ die Bedeutung 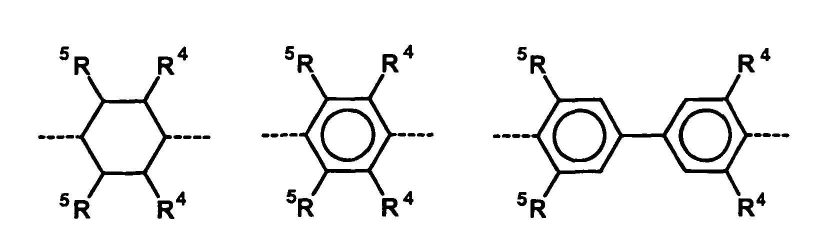 hat;
R² ein C₁ bis C₁₂-Alkylenrest, C₄-C₈-Cycloalkylenrest oder C₇ - bis C₁₅-Alkylenphenylenrest ist;
R³ Wasserstoff, ein C₁- bis C₅-Alkylrest oder ein C₁- bis C₅-O-Alkylrest ist; und
R⁴, R⁵ unabhängig voneinander jeweils für einen C₁- bis C₅-Alkylrest oder einen C₁- bis C₅-O-Alkylrest stehen.

2. Acrylesterphosphonsäure nach Anspruch 1, **dadurch gekennzeichnet, daß** eine oder mehrere der Variablen der Formel (I) unabhängig voneinander die folgenden Bedeutung haben:
n = 1,
R¹ = 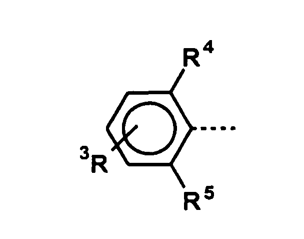
R² = ein C₁ bis C₆-Alkylenrest;
R³ = Wasserstoff, ein C₁- bis C₃-Alkylrest; und
R⁴, R⁵ = unabhängig voneinander ein C₁- bis C₃-Alkylrest.

3. Acrylesterphosphonsäure nach Anspruch 1 oder 2, **dadurch gekennzeichnet, daß** die Reste R², R³, R⁴ und/oder R⁵ unsubstituiert oder durch einen oder mehrere Substituenten ausgewählt aus der Gruppe Cl, Br, CH₃O, OH, COOH, CN, =O, =S, =NR⁶ oder -NR⁷-CO-C(=CH₂)CH₂-Y-R⁸-PO(OH)₂ substituiert sind, wobei R⁶ und R⁷ unabhängig voneinander jeweils für Wasserstoff, einen geradkettigen oder verzweigten C₁- bis C₁₀-Alkyl- oder C₆- bis C₁₀-Arylrest stehen und R⁸ ein geradkettiger oder verzweigter C₁- bis C₁₀-Alkylen- oder C₆- bis C₁₄-Arylen-Rest ist.

4. Zusammensetzung, **dadurch gekennzeichnet, daß** sie eine Acrylesterphosphonsäure nach einem der Ansprüche 1 bis 3 enthält.

5. Zusammensetzung nach Anspruch 4, **dadurch gekennzeichnet, daß** sie zusätzlich ein radikalisch polymerisierbares Monomer enthält.

6. Zusammensetzung nach Anspruch 5, **dadurch gekennzeichnet, daß** sie als radikalisch polymerisierbares Monomer ein Acrylamid und/oder ein Hydroxyalkylacrylamid enthält.

7. Zusammensetzung nach Anspruch 5 oder 6, **dadurch gekennzeichnet, daß** sie ein monofunktionelles und/oder ein mehrfunktionelles radikalisch polymerisierbares Monomer enthält.

8. Zusammensetzung nach Anspruch 7, **dadurch gekennzeichnet, daß** sie als monofunktionelles radikalisch polymerisierbares Monomer ein oder mehrere hydrolysestabile Mono (meth) acrylate, Mesitylmethacrylat, ein oder mehrere 2-(Alkoxymethyl)acrylsäuren, 2-(Ethoxymethyl)acrylsäure, 2-(Hydroxymethyl)acrylsäure, ein oder mehrere N-mono- oder N-disubstitiuierte Acrylamide, N-Ethylacrylamid, N,N-Dimethacrylamid, N- (2-Hydroxyethyl)acrylamid, N-(2-Hydroxyethyl)-N-methylacrylamid, ein oder mehrere. N-monosubstituierte Methacrylamide, N-Ethylmethacrylamid, N- (2-Hydroxyethyl)methacrylamid, N-Vinylpyrrolidon, Allylether oder eine Mischung von zwei oder mehr dieser Monomere enthält.

9. Zusammensetzung nach Anspruch 7 oder 8, **dadurch gekennzeichnet, daß** sie als mehrfunktionelles radikalisch polymerisierbares Monomer ein oder mehrere Urethane aus 2-(Hydroxymethyl)acrylsäure und Diisocyanate, 2,2,4-Trimethylhexamethylendiisocyanat, Isophorondiisocyanat, ein oder mehrere vernetzende Pyrrolidone, 1,6-Bis(3-vinyl-2-pyrrolidonyl)-hexan, ein oder mehrere Bisacrylamide, Methylenbisacrylamid, Ethylenbisacrylamid, ein oder mehrere Bis (meth) acrylamide, N,N'-Diethyl-1,3-bis(acrylamido)-propan, 1,3-Bis(methacrylamido)-propan, 1,4-Bis(acrylamido)-butan, 1,4-Bis(acryloyl)-piperazin oder eine Mischung aus zwei oder mehr dieser Monomere enthält.

10. Zusammensetzung nach einem der Ansprüche 4 bis 9, **dadurch gekennzeichnet, daß** sie zusätzlich einen Initiator für die radikalische Polymerisation enthält.

11. Zusammensetzung nach einem der Ansprüche 4 bis 10, **dadurch gekennzeichnet, daß** sie zusätzlich Füllstoff enthält.

12. Zusammensetzung nach einem der Ansprüche 4 bis 10, **dadurch gekennzeichnet, daß** sie zusätzlich Lösungsmittel enthält.

13. Zusammensetzung nach einem der Ansprüche 4 bis 12, **dadurch gekennzeichnet, daß** sie zusätzlich ein (Meth)acrylamidoalkyl-dihydrogenphosphat enthält.

14. Zusammensetzung nach einem der Ansprüche 4 bis 13, **dadurch gekennzeichnet, daß** sie
a) 0,5 bis 70 Gew.-% Acrylesterphosphonsäure nach Anspruch 1 oder 2;
b) 0,01 bis 15 Gew.-% Initiator für die radikalische Polymerisation;
c) 0 bis 80 Gew.-% radikalisch polymerisierbares Monomer;
d) 0 bis 95 Gew.-% Lösungsmittel;
e) 0 bis 50 Gew.-%, (Meth)acrylamidoalkyldihydrogenphosphat,
und/oder
f) 0 bis 75 Gew.-% Füllstoff enthält.

15. Verwendung einer Zusammensetzung gemäß einem der Ansprüche 4 bis 14 als Dentalmaterial.

16. Verwendung nach Anspruch 15 als Zement oder Adhäsiv.

17. Verwendung einer Acrylesterphosphonsäure gemäß einem der Ansprüche 1 bis 3 zur Herstellung eines Dentalmaterials.

## Claims

1. Acrylic ester phosphonic acid of the general Formula (I), stereoisomers thereof and their mixtures, 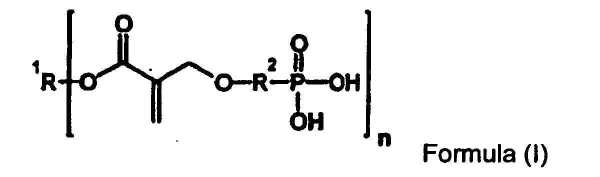 in which
n is 1 or 2,
with the proviso that
for n = 1, R¹ has the meaning 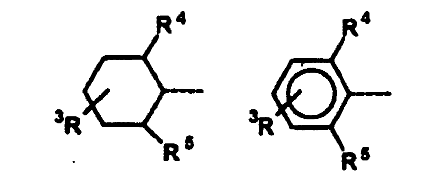 and for n = 2, R¹ has the meaning 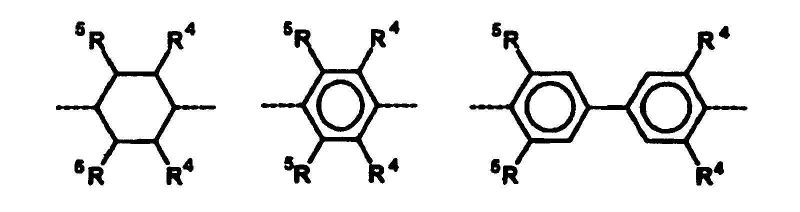 R² is a C₁ to C₁₂ alkylene radical, C₄-C₈ cycloalkylene radical or C₇-C₁₅ alkylene phenylene radical;
R³ is hydrogen, a C₁ to C₅ alkyl radical or a C₁ to C₅ O-alkyl radical; and
R⁴, R⁵ independently of each other, each stand for a C₁ to C₅ alkyl radical or a C₁ to C₅ O-alkyl radical.

2. Acrylic ester phosphonic acid according to Claim 1, **characterised in that** one or more of the variables of Formula (I), independently of each other, have the following meaning:
n = 1,
R¹ = 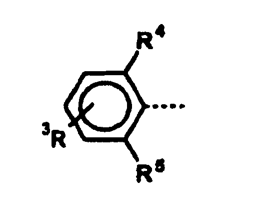
R² = a C₁ to C₆ alkylene radical;
R³ = hydrogen, a C₁ to C₃ alkyl radical; and
R⁴, R⁵ = independently of each other, a C₁ to C₃ alkyl radical.

3. Acrylic ester phosphonic acid according to Claim 1 or 2, **characterised in that** the radicals R², R³, R⁴ and/or R⁵ are unsubstituted or substituted by one or more substituents selected from the group Cl, Br, CH₃O, OH, COOH, CN, =O, =S, =NR⁶ or -NR⁷-CO-C(=CH₂)CH₂-Y-R⁸-PO(OH)₂, wherein R⁶ and R⁷, independently of each other, each stand for hydrogen, a straight-chained or branched C₁ to C₁₀ alkyl or C₆ to C₁₀ aryl radical and R⁸ is a straight-chained or branched C₁ to C₁₀ alkylene or C₆ to C₁₄ arylene radical.

4. Composition, **characterised in that** it comprises an acrylic ester phosphonic acid according to one of Claims 1 to 3.

5. Composition according to Claim 4, **characterised in that** it additionally comprises a radically polymerisable monomer.

6. Composition according to Claim 5, **characterised in that** it comprises an acrylamide and/or a hydroxyalkyl acrylamide as the radically polymerisable monomer.

7. Composition according to Claim 5 or 6, **characterised in that** it comprises a monofunctional and/or a multifunctional radically polymerisable monomer.

8. Composition according to Claim 7, **characterised in that** it comprises as a monofunctional radically polymerisable monomer one or more hydrolytically stable mono(meth)acrylates, mesityl methacrylate, one or more 2-(alkoxymethyl)acrylic acids, 2-(ethoxymethyl)acrylic acid, 2-(hydroxymethyl)acrylic acid, one or more N-mono- or N-disubstituted acrylamides, N-ethylacrylamide, N,N-dimethacrylamide, N-(2-hydroxyethyl)acrylamide, N-(2-hydroxyethyl)-N-methyl-acrylamide, one or more N-monosubstituted methacrylamides, N-ethylmethacrylamide, N-(2-hydroxyethyl)methacrylamide, N-vinyl pyrrolidone, allyl ether or a mixture of two or more of these monomers.

9. Composition according to Claim 7 or 8, **characterised in that** it comprises as a multifunctional radically polymerisable monomer one or more urethanes from 2-(hydroxymethyl)acrylic acid and diisocyanates, 2,2,4-trimethylhexamethylene diisocyanate, isophorone diisocyanate, one or more crosslinking pyrrolidones, 1,6-bis(3-vinyl-2-pyrrolidonyl)hexane, one or more bisacrylamides, methylene bisacrylamide, ethylene bisacrylamide, one or more bis(meth)acrylamides, N,N'-diethyl-1,3-bis(acrylamido)propane, 1,3-bis(methacrylamido)propane, 1,4-bis(acrylamido)butane, 1,4-bis(acryloyl) piperazine or a mixture of two or more of these monomers.

10. Composition according to one of Claims 4 to 9, **characterised in that** it additionally comprises an initiator for radical polymerisation.

11. Composition according to one of Claims 4 to 10, **characterised in that** it additionally comprises a filler.

12. Composition according to one of Claims 4 to 10, **characterised in that** it additionally comprises solvent.

13. Composition according to one of Claims 4 to 12, **characterised in that** it additionally comprises a (meth)acrylamidoalkyl dihydrogen phosphate.

14. Composition according to one of Claims 4 to 13, **characterised in that** it comprises
a) 0.5 to 70 wt.% acrylic ester phosphonic acid according to Claim 1 or 2;
b) 0.01 to 15 wt.% initiator for radical polymerisation;
c) 0 to 80 wt.% radically polymerisable monomer;
d) 0 to 95 wt.% solvent;
e) 0 to 50 wt.%, (meth)acrylamidoalkyl dihydrogen phosphate, and/or
f) 0 to 75 wt.% filler.

15. Use of a composition according to one of Claims 4 to 14 as dental material.

16. Use according to Claim 15 as cement or adhesive.

17. Use of an acrylic ester phosphonic acid according to one of Claims 1 to 3 for the preparation of a dental material.

## Revendications

1. Acide acryloesterphosphonique de formule générale (I), ses stéréo-isomères et mélanges de ceux-ci, 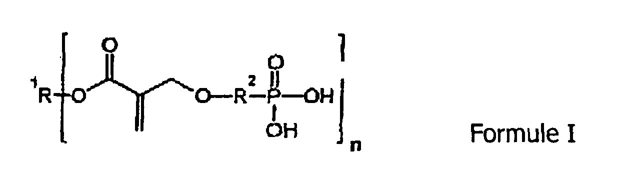 dans laquelle
n est 1 ou 2,
étant entendu que
pour n = 1, R¹ a la signification 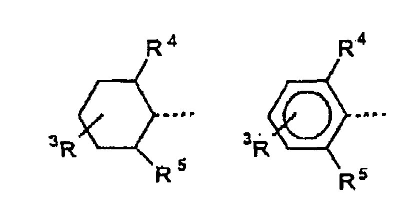 et pour n = 2, R¹ a la signification 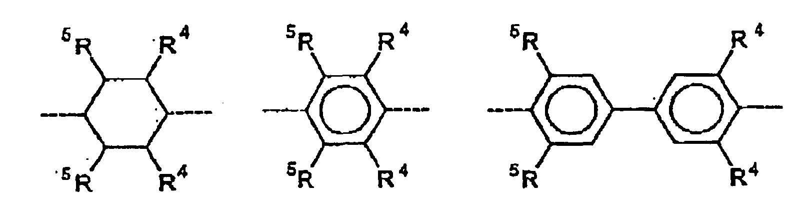
R² est un radical alkylène en C₁-C₁₂, un radical cycloalkylène en C₄-C₈ ou un radical alkylènephénylène en C₇-C₁₅ ;
R³ est une atome d'hydrogène, un radical alkyle en C₁-C₅ ou un radical O-alkyle en C₁-C₅ ; et
R⁴, R⁵ représentent chacun, indépendamment l'un de l'autre, un radical alkyle en C₁-C₅ ou un radical O-alkyle en C₁-C₅.

2. Acide acryloesterphosphonique selon la revendication 1, **caractérisé en ce qu'**une ou plusieurs des variables de la formule (I) ont, indépendamment les unes des autres, la signification suivante :
n = 1,
R¹ = 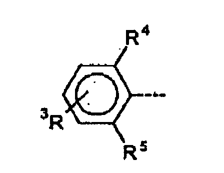
R² = un radical alkylène en C₁ à C₆ ;
R³ = un atome d'hydrogène, un radical alkyle en C₁-C₃ ; et
R⁴, R⁵ représentent chacun, indépendamment l'un de l'autre, un radical alkyle en C₁-C₃.

3. Acide acryloesterphosphonique selon la revendication 1 ou 2, **caractérisé en ce que** les radicaux R², R³, R⁴ et/ou R⁵ sont non substitués ou portent un ou plusieurs substituants choisis dans le groupe constitué par Cl, Br, CH₃O, OH, COOH, CN, =O, =S, =NR⁶ et -NR⁷-CO-C(=CH₂)CH₂-Y-R⁸-PO(OH)₂, R⁶ et R⁷ représentant chacun, indépendamment l'un de l'autre, un atome d'hydrogène, un radical alkyle en C₁-C₁₀ à chaîne droite ou ramifiée ou aryle en C₆-C₁₀, et R⁸ est un radical alkylène en C₁-C₁₀ à chaîne droite ou ramifiée ou arylène en C₆-C₁₄.

4. Composition, **caractérisée en ce qu'**elle contient un acide acryloesterphosphonique selon l'une quelconque des revendications 1 à 3.

5. Composition selon la revendication 4, **caractérisée en ce qu'**elle contient en outre un monomère apte à la polymérisation radicalaire.

6. Composition selon la revendication 5, **caractérisée en ce qu'**elle contient en tant que monomère apte à la polymérisation radicalaire un acrylamide et/ou un hydroxyalkylacrylamide.

7. Composition selon la revendication 5 ou 6, **caractérisée en ce qu'**elle contient un monomère monofonctionnel apte à la polymérisation radicalaire et/ou un monomère polyfonctionnel apte à la polymérisation radicalaire.

8. Composition selon la revendication 7, **caractérisée en ce qu'**elle contient en tant que monomère monofonctionnel apte à la polymérisation radicalaire un ou plusieurs mono(méth)acrylates stables vis-à-vis de l'hydrolyse, du méthacrylate de mésityle, un ou plusieurs acides 2-(alcoxyméthyl)acryliques, de l'acide 2-(éthoxyméthyl)acrylique, de l'acide 2-(hydroxyméthyl)acrylique, un ou plusieurs acrylamides N-mono- ou N-disubstitués, du N-éthylacrylamide, du N,N-diméthacrylamide, du N-(2-hydroxyéthyl)acrylamide, du N-(2-hydroxyéthyl)-N-méthylacrylamide, un ou plusieurs méthacrylamides N-monosubsitués, du N-éthylméthacrylamide, du N-(2-hydroxyéthyl)méthacrylamide, de la N-vinylpyrrolidone, des éthers allyliques ou un mélange de deux ou plus de deux de ces monomères.

9. Composition selon la revendication 7 ou 8, **caractérisée en ce qu'**elle contient, en tant que monomère polyfonctionnel apte à la polymérisation radicalaire un ou plusieurs uréthannes à base d'acide 2-(hydroxyméthyl)acrylique et de düsocyanates, du 2,2,4-triméthylhexaméthylène-düsocyanate, de l'isophorone-diisocyanate, une ou plusieurs pyrrolidones réticulables, du 1,6-bis(3-vinyl-2-pyrrolidonyl)hexane, un ou plusieurs bis-acrylamides, du méthylène-bisacrylamide, de l'éthylène-bis-acrylamide, un ou plusieurs bis(méth)acrylamides, du N,N'-diéthyl-1,3-bis(acrylamido)propane, du 1,3-bis(méthacrylamido)propane, du 1,4-bis(acrylamido)butane, de la 1,4-bis(acryloyl)pipérazine ou un mélange de deux ou plus de deux de ces monomères.

10. Composition selon l'une quelconque des revendications 4 à 9, **caractérisée en ce qu'**elle contient additionnellement un amorceur pour la polymérisation radicalaire.

11. Composition selon l'une quelconque des revendications 4 à 10, **caractérisée en ce qu'**elle contient en outre une charge.

12. Composition selon l'une quelconque des revendications 4 à 10, **caractérisée en ce qu'**elle contient en outre un solvant.

13. Composition selon l'une quelconque des revendications 4 à 12, **caractérisée en ce qu'**elle contient en outre un dihydrogénophosphate de (méth)acrylamidoalkyle.

14. Composition selon l'une quelconque des revendications 4 à 13, **caractérisée en ce qu'**elle contient
a) 0,5 à 70 % en poids d'acide acryloesterphosphonique selon la revendication 1 ou 2 ;
b) 0,01 à 15 % en poids d'amorceur pour la polymérisation radicalaire ;
c) 0 à 80 % en poids de monomère apte à la polymérisation radicalaire ;
d) 0 à 95 % en poids de solvant ;
e) 0 à 50 % en poids de dihydrogénophosphate de (méth)acrylamidoalkyle ;
et/ou
f) 0 à 75 % en poids de charge.

15. Utilisation d'une composition selon l'une quelconque des revendications 4 à 14 en tant que matériau dentaire.

16. Utilisation selon la revendication 15, en tant que colle ou adhésif.

17. Utilisation d'un acide acryloesterphosphonique selon l'une quelconque des revendications 1 à 3, pour la préparation d'un matériau dentaire.
